# FASCICULE DE BREVET EUROPEEN

(11) **EP 0 950 052 B1**
(45) Date de publication et mention de la délivrance du brevet: **08.09.2004**
(21) Numéro de dépôt: 97952988.0
(22) Date de dépôt: 23.12.1997
(51) Int. Cl.: C07D 211/70, A61K 31/44, C07C 17/16, C07C 29/147, C07C 22/04, C07C 33/18

(54) **PROCEDE POUR LA PREPARATION D'UN DERIVE DE TETRAHYDROPYRIDINE**
VERFAHREN ZUR HERSTELLUNG EINES TETRAHYDROPYRIDINDERIVATES
METHOD FOR PREPARING A TETRAHYDROPYRIDIN DERIVATIVE

(30) Priorité: 23.12.1996 FR 9615906
(43) Date de publication de la demande: 20.10.1999
(73) Titulaire: SANOFI-SYNTHELABO, 75013 Paris (FR)
(72) Inventeur: BUJ, Michel, F-34170 Castelnau le Lez (FR); FILHOL, Robert, Rue des Avant-Mont F-34080 Montpellier (FR)
(74) Mandataire: Gillard, Marie-Louise
(86) Numéro de dépôt international: PCT/FR1997/002395
(87) Numéro de publication internationale: WO 1998/028273

(56) Documents cités:
- GB-A- 2 018 136
- CAMPAIGNE E. ET AL: "Sulfur heterocycles from the ring closure of bisarylalkyl disulfides" JOURNAL OF ORGANIC CHEMISTRY., vol. 29, 1964, pages 2372-2378, XP002247518 AMERICAN CHEMICAL SOCIETY. EASTON., US ISSN: 0022-3263
- GILBERT, ANDREW ET AL: "Inter- and intramolecular photocycloaddition of enol ethers to naphthalene" JOURNAL OF THE CHEMICAL SOCIETY, PERKIN TRANSACTIONS 1: ORGANIC AND BIO-ORGANIC CHEMISTRY (1972-1999) (1988), (1), 31-6 , XP002247519
- CAGNIANT P. ET AL: "Contribution à l' étude des hétérocycles soufrés condensés-XV. Recherches dans le domaine des sulfures semiaromatiques tricycliques: Synthèses et propriétés du naphto-2',1'-4,5 thia-I cycloheptène" BULLETIN DE LA SOCIETE CHIMIQUE DE FRANCE., 1961, pages 1934-1937, XP002247520 SOCIETE FRANCAISE DE CHIMIE. PARIS., FR ISSN: 0037-8968

## Description

La présente invention concerne un procédé pour la préparation de la 1-[2-(2-naphtyl)éthyl]-4-(3-trifluorométhylphényl)-1,2,3,6-tétrahydropyridine et de ses sels pharmaceutiquement acceptables.

La 1-[2-(2-naphtyl)éthyl]-4-(3-trifluorométhylphényl)-1,2,3,6-tétrahydropyridine, ci-après désignée par son numéro de code SR 57746, et ses sels pharmaceutiquement acceptables ont été décrits pour la première fois dans EP 0 101 381 comme étant des agent anorexigènes et, par la suite, comme anti-anxiodépresseurs (US 5,026,716), anticonstipants (US 5,109,005), neurotrophiques (US 5,270,320), anti-radicaux libres (US 5,292,745) et cardioprotecteurs (US 5,378,709).

Le document EP 0 101 381 décrit une série de 1-(hétéro)aralkyl-4-(3-trifluorométhylphényl)-1,2,3,6-tétrahydropyridines préparées par condensation de la 4-(3-trifluorométhylphényl)-1,2,3,6-tétrahydropyridine :
- soit avec un (hétéro)aralkylhalogénure, notamment chlorure, bromure et iodure ou un dérivé analogue contenant un groupe partant électrophile, tel que le groupe méthanesulfonyloxy ou p-toluènesulfonyloxy;
- soit, lorsque le groupe alkylène est linéaire, avec un halogénure d'un acide (hétéro)aralcanoique, ladite condensation étant suivie par la réduction de l'amide ainsi obtenu.

Selon le document cité ci-dessus, la 1-[2-(2-naphtyl)éthyl]-4-(3-trifluorométhylphényl)-1,2,3,6-tétrahydropyridine est préparée sous forme de chlorhydrate par réaction de la 4-(3-trifluorométhylphényl)-1,3,3,6-tétrahydropyridine avec un chlorure de 2-naphtylacétyle et réduction du produit ainsi obtenu par de l'hydrure de lithium et d'aluminium. Ce procédé se déroule de façon satisfaisante dans la première étape, mais la réduction suivante comporte une diminution des rendements causée par l'attaque du groupe trifluorométhyle de la part de l'agent réducteur, comme le démontre le rendement de 42,73% de la théorie obtenu dans la préparation décrite.

Le même document décrit la préparation du chlorhydrate de la 1-[2-(2-naphtyl)éthyl]-4-(3-trifluorométhylphényl)-1,2,3,6-tétrahydropyridine par condensation de la 4-(3-trifluorométhylphényl)-1,2,3,6-tétrahydropyridine avec le 2-(2-chloroéthyl)naphtalène en présence de triéthylamine, mais aucune indication n'est donnée au sujet des rendements obtenus. Indépendamment des rendements qui peuvent être obtenus par cette alkylation, l'utilisation du 2-(2-chloroéthyl)naphtalène comporte cependant des problèmes liés à la fabrication de cet intermédiaire, qui prévoit le chauffage du 2-(2-naphtyl)éthanol dans le chlorure de thionyle. Cette réaction donne des rendements très faibles en dérivé chloré d'une part parce que le naphtyléthanol ne réagit pas complètement et, d'autre part, parce que la réaction donne des quantités variables - selon les conditions opératoires - de 2-vinylnaphtalène.

Des rendements meilleurs (83,9%) sont obtenus en faisant réagir le 2-naphtalènéthanol avec le chlorure de thionyle dans de l'éther en présence de pyridine (J. Am. Chem Soc., 1982, 104(19):5171), mais une réaction de ce type doit être suivie avec la plus grande attention et, notamment, elle est difficilement exploitable au niveau industriel.

En outre, il a été constaté qu'en faisant réagir le 2-(2-chloroéthyl)naphtalène sur la 4-(3-trifluorométhylphényl)-1,2,3,6-tétrahydropyridine dans les conditions décrites dans EP 0 101 381, à savoir dans l'éthanol au reflux pendant 20-24 heures, on obtient le chlorhydrate de 1-[2-(2-naphtyl)éthyl]-4-(3-trifluorométhylphényl)-1,3,6-tétrahydropyridine avec des rendements très faibles.

Il a été maintenant trouvé qu'en faisant réagir la 4-(3-trifluorométhylphényl)-1,2,3,6-tétrahydropyridine, ou l'un de ses sels, avec le 2-(2-bromoéthyl)naphtalène, la 1-[2-(2-naphtyl)éthyl]-4-(3-trifluorométhylphényl)-1,2,3,6-tétrahydropyridine et ses sels peuvent être obtenus avec des rendements bien supérieurs à ceux obtenus selon EP 0 101 381.

II a été également trouvé que les produits ainsi obtenus sont plus purs que le chlorhydrate de 1-[2-(2-naphtyl)éthyl]-4-(3-trifluorométhylphényl)-1,2,3,6-tétrahydropyridine préparé en utilisant le 2-(2-chloroéthyl)naphtaléne selon EP 0 101 38l parce qu'ils sont pratiquement exempts de dérivés vinylés. De plus, le 2-(2-bromoéthyl)naphtalène utilisé comme réactif peut être préparé très facilement et avec des rendements supérieurs à 90% à partir du 2-naphtyléthanol et de l'acide bromhydrique et le produit obtenu ne contient pas de 2-vinylnaphtalène ou en contient en quantités qui ne dépassent pas 0,1%.

Il a été enfin trouvé que le 2-(2-bromoéthyl)naphtalène peut être obtenu directement à partir de l'acide naphtylacétique sans isoler le 2-naphtyléthanol avec des rendements excellents, même supérieurs à 80%.

Ainsi, selon un de ses aspects, la présente invention a pour objet un procédé pour la préparation de la 1-[2-(2-naphtyl)éthyl]-4-(3-trifluorométhylphényl)-1,2,3,6-tétrahydropyridine de formule (I) 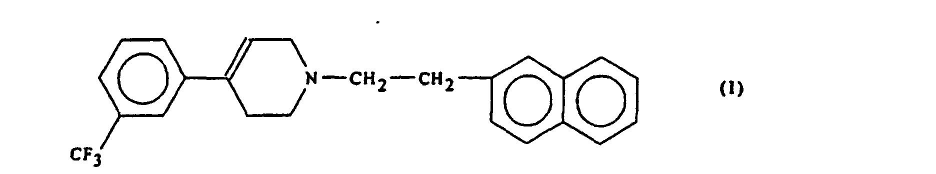 et de ses sels pharmaceutiquement acceptables, caractérisé en ce qu'on traite la 4-(3-trifluorométhylphényl)-1,2,3,6-tétrahydropyridine de formule (II) 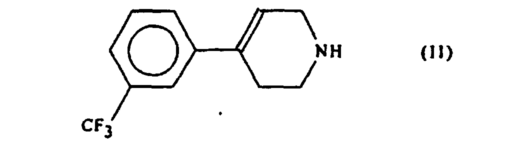 ou l'un de ses sels, avec le 2-(2-bromoéthyl)naphtalène de formule (III) 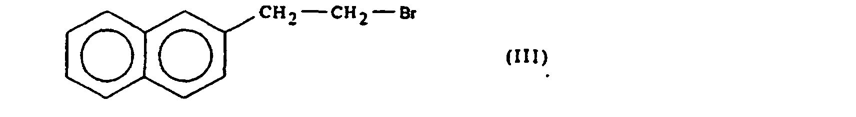 en présence d'une base et à une température comprise entre 24°C et la température de reflux du solvant employé.

Comme composé de formule (II) de départ, on peut indifféremment utiliser la 4-(3-trifluorométhylphényl)-1,2,3,6-tétrahydropyridine de formule (II) sous forme de base libre ou d'un de ses sels, dans ce dernier cas sous forme de chlorhydrate de préférence.

Le solvant peut être protique ou aprotique, de préférence polaire, comme par exemple un alcool en C₁-C₃, tel que le méthanol ou l'éthanol, seul ou en mélange avec de l'eau, l'acétonitrile ou une cétone comme par exemple l'acétone ou la méthylisobutylcétone.

Selon un mode opératoire avantageux, la réaction est conduite à partir du composé de formule (II) sous forme de chlorhydrate. Plus avantageusement, on fait réagir le chlorhydrate de 4-(3-trifluorométhylphényl)-1,2,3,6-tétrahydropyridine avec le 2-(2-bromoéthyl)naphtalène dans un solvant protique ou aprotique polaire, tels que ceux cités ci-dessus, au reflux et en présence d'une base.

Selon un mode opératoire particulièrement avantageux, la réaction entre le composé (II) sous forme de chlorhydrate et le composé (III) est effectuée dans un solvant choisi parmi les mélanges éthanol-eau, l'acétonitrile et l'acétone à la température de reflux en présence d'une base choisie parmi les hydroxydes et les carbonates alcalins.

Dans ces conditions particulièrement avantageuses, la réaction est complète après 3-8 heures de chauffage et le composé de formule (I) est isolé selon les techniques conventionnelles, par exemple soit par simple filtration de la base libre ainsi obtenue, soit par traitement avec une solution d'un acide pour récupérer le sel correspondant susceptible d'être ensuite neutralisé pour obtenir la base libre, qui peut à son tour être transformée en l'un de ses sels pharmaceutiquement acceptables. Les rendements en produit final, dans ces conditions, sont très satisfaisants et peuvent atteindre 80-90%.

Selon cette méthode, le composé de formule (I) ainsi obtenu a une pureté très élevée et, notamment, contrairement à un produit obtenu à partir du chloroéthylnaphtalène selon EP 0 101 381, ne contient pas de quantité détectable de dérivé vinylé.

Comme il a été mentionné ci-dessus, l'utilisation du 2-(2-chloroéthyl)naphtalène comporte le désavantage que la préparation du produit par réaction du 2-(2-naphtyl)éthanol avec le chlorure de thionyle a lieu avec de très bas rendements aussi à cause de la formation de quantités non négligeables de 2-vinylnaphtalène qui sont éliminées pendant l'isolement du produit souhaité.

Ces réactions secondaires ne se produisent pas lorsque le 2-(2-bromoéthyl)naphtalène est préparé par un procédé caractérisé en ce qu'on réduit l'acide 2-naphtylacétique et on traite ensuite le 2-(2-naphtyl)éthanol brut ainsi obtenu avec l'acide bromhydrique concentré.

La réduction est effectuée de préférence avec un hydrure de bore ou d'aluminium, éventuellement mixte, choisi parmi l'hydrure de lithium et d'aluminium, l'hydrure de sodium bis-(2-méthoxyethoxy)aluminium et le diborane, dans un solvant organique de type éther comme le méthyl t-butyl éther, le dioxane ou le tétrahydrofurane.

Selon un mode opératoire préférentiel, on traite l'acide naphtylacétique avec l'hydrure de lithium et d'aluminium dans le tétrahydrofurane, avantageusement à une température inférieure à 20 °C et, après élimination des sels, par exemple par addition d'un hydroxyde alcalin et élimination de l'insoluble par filtration, on évapore le tétrahydrofurane et on traite le résidu constitué par le 2-(2-naphtyl)éthanol brut, avec de l'acide bromhydrique concentré (47-48%). On isole ainsi le 2-(2-bromoéthyl)naphtalène avec des rendements très élevés, même supérieurs à 80% par rapport à l'acide napthylacétique de départ.

De plus, le 2-(2-bromoéthyl)naphtalène ainsi obtenu est très pur car il contient moins de 0,1% de dérivé vinylé.

Selon un aspect préférentiel, la présente invention concerne notamment un procédé pour la préparation de la 1-[2-(2-naphtyl)éthyl]-4-(3-trifluorométhylphényl)-1,2,3,6-tétrahydropyridine et de ses sels pharmaceutiquement acceptables, caractérisé en ce que
(a) on réduit l'acide 2-naphtylacétique en 2-(2-naphtyl)éthanol et on fait réagir le produit ainsi obtenu, sans le purifier, avec de l'acide bromhydrique concentré; puis
(b) on traite le 2-(2-bromoéthyl)naphtalène ainsi obtenu avec la 4-(3-trifluorométhylphényl)-1,2,3,6-tétrahydropyridine ou l'un de ses sels en présence d'une base; et
(c) on isole la 1-[2-(2-naphtyl)éthyl]-4-(3-trifluorométhylphényl)-1,2,3,6-tétrahydropyridine soit sous forme de base qu'on transforme éventuellement en l'un de ses sels pharmaceutiquement acceptables, soit sous forme d'un de ses sels qu'on neutralise éventuellement pour obtenir la base libre.

L'étape (a) est conduite de préférence en utilisant comme agent réducteur un hydrure de bore ou d'aluminium, éventuellement mixte, l'hydrure de lithium et d'aluminium, l'hydrure de sodium bis-(2-méthoxyéthoxy)aluminium et le diborane étant particulièrement avantageux.

Le diborane est normalement utilisé sous forme d'un de ses complexes, par exemple avec le diméthylsulfure ou avec le tétrahydrofurane. Ce complexe peut être facilement généré in situ.

L'hydrure de lithium et d'aluminium est l'agent réducteur préféré.

De préférence la réduction est effectuée dans le tétrahydrofurane, à une température inférieure à 20°C lorsque l'agent réducteur est l'hydrure de lithium et d'aluminium ou bien au reflux lorsque l'agent réducteur utilisé est le diborane.

Lorsque la réduction est terminée, l'agent réducteur est détruit selon les méthodes conventionnelles, par exemple par une base comme l'hydroxyde de sodium, et les sels sont éliminés avec la phase aqueuse et, après évaporation du solvant, le 2-(2-naphtyl)éthanol brut ainsi obtenu est directement soumis à la réaction avec de l'acide bromhydrique concentré. Cette bromuration est effectuée au reflux et elle est complète après 4-8 heures de chauffage.

Le 2-(2-bromoéthyl)naphtalène, ainsi obtenu avec un rendement supérieur à 80% par rapport à l'acide naphtylacétique, est isolé par simple filtration et cristallisé dans l'isopropanol. Il est pur et ne contient pas de quantité détectable de dérivé vinylé.

L'étape (b), à savoir la réaction du 2-(2-bromoéthyl)naphtalène ainsi obtenu et la 4-(3-trifluorométhylphényl)-1,2,3,6-tétrahydropyridine ou, de préférence, son chlorhydrate, est avantageusement conduite dans un solvant protique ou aprotique polaire. De préférence elle se déroule avec de très bons rendements et sans formation de dérivés vinylés dans un solvant choisi parmi l'acétonitrile, l'acétone et les mélanges eau-éthanol au reflux en présence d'une base choisie parmi les hydroxydes et les carbonates alcalins.

Selon un mode opératoire particulièrement avantageux, on traite le 2-(2-bromoéthyl)naphtalène avec le chlorhydrate de 4-(3-trifluorométhylphényl)-1,2,3,6-tétrahydropyridine dans un mélange eau/éthanol de 2/1 à 1/1 (v/v) en présence d'un hydroxyde alcalin, notamment d'hydroxyde de sodium, au reflux et, en général, la réaction est complète après 4-6 heures.

Dans l'étape (c), la 1-[2-(2-naphtyl)éthyl]-4-(3-tnfluorométhylphényl)-1,2,3,6-tétrahydropyridine, obtenue à l'étape (b) avec un rendement qui peut atteindre 90% de la théorie dans les conditions du mode opératoire particulièrement avantageux illustré ci-dessus, est isolée soit sous forme de base libre soit sous forme d'un de ses sels.

Selon le mode opératoire particulièrement avantageux illustré ci-dessus, l'utilisation du solvant hydroalcoolique permet, lors du refroidissement du mélange réactionnel, la précipitation de la 1-[2-(2-naphtyl)éthyl]-4-(3-trifluorométhylphényl)-1,2,3,6-tétrahydropyridine base qui peut être isolée par simple filtration, lavée à l'eau et séchée.

La base libre ainsi obtenue peut être transformée dans un de ses sels pharmaceutiquement acceptables par traitement avec une solution de l'acide convenable dans un solvant organique ou aqueux-organique et cristallisation.

Le sel pharmaceutiquement acceptable préféré, le chlorhydrate, peut être obtenu par réaction de la base avec une solution d'acide chlorhydrique dans l'éthanol et cristallisation dans un solvant approprié, tel que l'éthanol, les mélanges éthanol-eau, l'acétone, la méthyléthylcétone, l'acétate d'éthyle et leurs mélanges avec l'eau, le mélange éthanol-acide chlorhydrique ou le diméthylsulfoxyde.

La 1-[2-(2-naphtyl)éthyl]-4-(3-trifluorométhylphényl)-1,2,3,6-tétrahydropyridine peut être également isolée sous forme d'un de ses sels pharmaceutiquement acceptables ou non, d'où la base libre peut être libérée par neutralisation avec, par exemple, un hydroxyde alcalin et, éventuellement, transformée dans un sel pharmaceutiquement acceptable comme illustré ci-dessus. De préférence, la 1-[2-(2-naphtyl)éthyl]-4-(3-trifluorométhylphényl)-1,2,3,6-tétrahydropyridine est isolée sous forme de son sel préféré, le chlorhydrate qui est recristallisé dans des solvants appropriés, tels que ceux cités ci-dessus.

Les exemples suivants illustrent l'invention.

### EXEMPLE 1

On chauffe un mélange de 12,5 g de 2-(2-bromoéthyl)naphtalène, 14 g de chlorhydrate de 4-(3-trifluorométhylphényl)-1,2,3,6-tétrahydropyridine, 4,34 g d'hydroxyde de sodium, 135 ml d'eau et 95 ml d'éthanol 95% pendant 5 heures au reflux, puis on laisse refroidir le mélange réactionnel pendant une nuit à la température ambiante, puis on filtre, on lave à l'eau le produit ainsi isolé et on le sèche sous vide à 50 °C. On obtient ainsi la 1-[2-(2-naphtyl)éthyl]-4-(3-trifluorométhylphényl)-1,2,3,6-tétrahydropyridine base avec un rendement de 90 % calculé sur le chlorhydrate de 4-(3-trifluorométhylphényi)-1,2,3,6-tétrahydropyridine de départ.

### EXEMPLE 2

On chauffe un mélange de 6,25 g de 2-(2-bromoéthyl)naphtalène, 7 g de chlorhydrate de 4-(3-trifluorométhylphényl)-1,2,3,6-tétrahydropyridine, 3,75 g de carbonate de potassium, 100 ml d'acétone pendant 4 heures au reflux, puis on laisse refroidir le mélange réactionnel jusqu'à la température ambiante. Les sels formés sont filtrés et éliminés. Le solvant est évaporé et le résidu est repris avec une solution d'acide chlorhydrique dans de l'éthanol. On obtient ainsi le chlorhydrate de 1-[2-(2-naphtyl)éthyl]-4-(3-trifluorométhylphényl)-1,2,3,6-tétrahydropyridine qu'on cristallise dans l'éthanol. Rendement: 70% de la théorie sur le chlorhydrate de 4-(3-trifluorométhylphényl)-1,2,3,6-tétrahydropyridine de départ. Le produit obtenu est sous forme d'une fine poudre cristalline blanche ayant une pureté HPLC de 99,9%.

### EXEMPLE 3

En opérant dans les conditions décrites dans l'Exemple 2, par chauffage pendant trois heures au reflux dans l'acétonitrile, on isole un chlorhydrate de 1-[2-(2-naphtyl)éthyl]-4-(3-trifluorométhylphényl)-1,2,3,6-tétrahydropyridine de pureté HPLC de 99,9% avec un rendement de 80,1% calculé sur le chlorhydrate de 4-(3-trifluorométhylphényl)-1,2,3,6-tétrahydropyndine de départ.

### EXEMPLE 4

A une solution de 17,2 g de 1-[2-(2-naphtyl)éthyl]-4-(3-trifluorométhylphényl)-1,2,3,6-tétrahydropyridine base obtenue selon l'Exemple 1, dans 200 ml d'éthanol absolu on ajoute sous agitation 6,25 ml d'acide chlorhydrique concentré. Le mélange ainsi obtenu est chauffé au reflux pendant 90 minutes, puis la solution est d'abord filtrée à chaud et ensuite évaporée de façon à éliminer environ 100 ml de solvant. On ajoute au mélange 20 ml d'eau distillée et on porte la température de la solution à 75°C, puis on refroidit ladite solution jusqu'à 5°C à une vitesse de 10°C par heure. On maintient le mélange une heure environ à 5°C, puis on recueille le produit par filtration et on le lave avec un mélange de 32 ml d'éthanol absolu et 3 ml d'eau. On sèche le produit sous vide à 50°C pour obtenir le chlorhydrate de 1-[2-(2-naphtyl)éthyl]-4-(3-trifluorométhylphényl)-1,2,3,6-tétrahydropyridine.

### EXEMPLE 5

### a/ 2-(2-Bromoéthyl)naphtalène.

A un mélange de 27,5 l de tétrahydrofurane et 10 kg d'hydrure de lithium et d'aluminium on ajoute, à une température inférieure à 20°C, une solution de 27,8 kg d'acide 2-naphtylacétique dans 95 l de tétrahydrofurane. On refroidit à 0°C, puis on ajoute lentement d'abord 10 l d'eau, puis une solution de 1,5 kg d'hydroxyde de sodium dans 10 l d'eau et, enfin, 30 l d'eau. On élimine par filtration les sels qui se séparent, après les avoir lavés avec 160 l de tétrahydrofurane. Les solutions en tétrahydrofurane réunies sont évaporées et le résidu, constitué par du 2-(2-naphtyl)éthanol estimé à 24,5 kg, est traité avec 138 l d'acide bromhydrique concentré. On chauffe le mélange au reflux pendant 5 heures, on le laisse revenir à la température ambiante sous agitation puis on filtre le produit obtenu et on le lave à l'eau. On dissout le produit humide dans 147 l d'isopropanol au reflux, on élimine environ 75 l de solvant par distillation et on laisse refroidir le mélange pendant une nuit. On filtre le produit ainsi cristallisé, on le lave avec de l'isopropanol préalablement refroidi et on le sèche sous vide à 40°C. On obtient ainsi le 2-(2-bromoéthyl)naphtalène ne contenant pas de quantité détectable de dérivé vinylé. Rendement: 81% calculé sur l'acide naphtylacétique de départ.

### b/ 1-[2-(2 Naphtyl)éthyl]-4-(3-trifluorométhylphényl)1,2,3,6-tétrahydropyridine.

A un mélange de 4,34 kg d'hydroxyde de sodium, 135 l d'eau et 95 l d'éthanol 95% on ajoute 12,5 kg de 2-(2-bromoéthyl)naphtalène et 14 kg de chlorhydrate de 4-(3-trifluorométhylphényl)-1,2,3,6-tétrahydropyridine. On chauffe le mélange réactionnel au reflux pendant au moins 4 heures, puis on le laisse refroidir pendant une nuit jusqu'à la température ambiante, de façon à laisser précipiter le produit de réaction.

### c/ Isolement de la base.

Le précipité obtenu dans l'opération (b) est recueilli par filtration et lavé deux fois avec des portions de 14 l d'eau, puis on sèche le produit sous vide à environ 50°C. On obtient ainsi la 1-[2-(2-naphtyl)éthyl]-4-(3-trifluorométhylphényl)-1,2,3,6-tétrahydropyridine base. P.f. 129-131°C. Rendement global calculé sur l'acide 2-naphtylacétique de départ: 74,3%.

### EXEMPLE 6

Dans deux préparations différentes, on fait réagir la 4-(3-trifluorométhylphényl)-1,2,3,6-tétrahydropyridine, en présence de triéthylamine, avec le 2-(2-bromoéthyl)naphtalène (PREPARATION A) et, respectivement, avec le 2-(2-chloroéthyl)naphtalène (PREPARATION B), dans de l'éthanol à reflux pendant 20 heures. Les mélanges réactionnels des deux préparations ont été concentrés, le résidu a été repris dans de l'éther éthylique et la solution éthérée, filtrée, lavée à l'eau et séchée, a été évaporée. Le résidu, repris avec une solution d'acide chlorhydrique gazeux dans l'isopropanol, a donné un chlorhydrate de 1-[2-(2-naphtyl)éthyl]-4-(3-trifluorométhylphényl)-1,2,3,6-tétrahydropyridine qui a été cristallisé dans l'éthanol.
- PREPARATION A:: Rendement: 59,8%
Pureté HPLC: 99,9%
Teneur en vinyle estimée: non détectable
- PREPARATION B:: Rendement: 7,5%
Purété HPLC: 97,8%
Teneur en vinyle estimée: 2,1%

## Revendications

1. Procédé pour la préparation de la 1-[2-(2-naphtyl)éthyl]-4-(3-trifluorométhylphenyl)-1,2,3,6-tétrahydropyridine de formule (I) 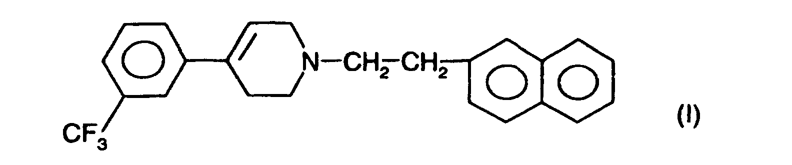 et de ses sels pharmaceutiquement acceptables, **caractérisé en ce qu'**on traite la 4-(3-trifluorométhylphényl)-1,2,3,6-tétrahydropyridine de formule (II) 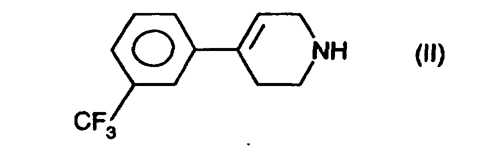 ou l'un de ses sels, avec le 2-(2-bromoéthyl)naphtalène de formule (III) 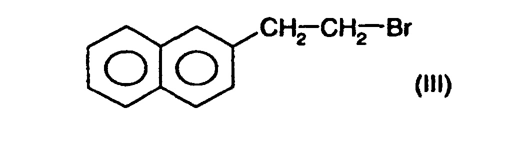 en présence d'une base et à une température comprise entre 20°C et la température de reflux du solvant employé.

2. Procédé selon la revendication 1, **caractérisé en ce que** le composé (II) est utilisé sous forme de chlorhydrate.

3. Procédé selon la revendication 2, **caractérisé en ce que** la réaction est conduite dans un solvant protique ou aprotique polaire au reflux.

4. Procédé selon la revendication 3, **caractérisé en ce que** la réaction est conduite dans un solvant choisi parmi l'acétone, l'acétonitrile et les mélanges eau-éthanol en présence d'une base choisie parmi les hydroxydes et les carbonates alcalins.

5. Procédé pour la préparation de la 1-[2-(2-naphtyl)éthyl]4-(3-trifluorométhylphényl)-1,2,3,6-tétrahydropyridine et de ses sels pharmaceutiquement acceptables, **caractérisé en ce que** :
(a) on réduit l'acide 2-naphtylacétique en 2-(2-naphtyl)éthanol et on fait réagir le produit ainsi obtenu, sans le purifier, avec de l'acide bromhydrique concentré ; puis
(b) on traite le 2-(2-bromoéthyl)naphtalène ainsi obtenu avec de la 4-(3-trifluorométhylphényl)-1,2,3,6-tétrahydropyridine ou l'un de ses sels ; et
(c) on isole la 1-[2-(2-naphtyl)éthyl]-4-(3-trifluorométhylphényl)-1,2,3,6-tétrahydropyridine soit sous forme de base que l'on transforme éventuellement en l'un de ses sels pharmaceutiquement acceptables, soit sous forme d'un de ses sels qu'on neutralise éventuellement pour obtenir la base libre.

6. Procédé selon la revendication 5, **caractérisé en ce que** dans l'étape (a) on utilise l'hydrure de lithium et d'aluminium en tant qu'agent réducteur.

7. Procédé selon la revendication 6, **caractérisé en ce que** la réduction est effectuée dans le tétrahydrofurane.

8. Procédé selon l'une quelconque des revendications 5 à 7, **caractérisé en ce que**, dans l'étape (b) on utilise le chlorhydrate de 4-(3-trifluorométhylphényl)-1,2,3,6-tétrahydropyridine.

9. Procédé selon. la revendication 8, **caractérisé en ce que** la réaction de l'étape (b) est conduite dans un solvant protique ou aprotique polaire en présence d'une base.

10. Procédé selon la revendication 9, **caractérisé en ce que** le solvant pratique ou aprotique polaire est choisi parmi l'acétonitrile, l'acétone et les mélanges éthanol-eau au reflux et la base est choisie parmi les hydroxydes et les carbonates alcalins.

11. Procédé selon la revendication 10, **caractérisé en ce que** l'étape (b) est conduite en traitant le 2-(2-bromoéthyl)naphtalène dans un mélange éthanol-eau de 2/1 à 1/1 (v/v) en présence d'un hydroxyde alcalin, au reflux.

12. Procédé selon la revendication 11, **caractérisé en ce que** l'on utilise l'hydroxyde de sodium en tant qu'hydroxyde alcalin.

13. Procédé selon la revendication 11 ou 12, **caractérisé en ce que**, dans l'étape (c), on isole par filtration la 1-[2|(2-naphtyl)éthyl]-4-(3-trifluorométhylphényl)-1,2,3,6-tétrahydropyridine base qui est éventuellement transformée en l'un de ses sels pharmaceutiquement acceptables.

14. Procédé selon la revendication 13, caractérisé en que la base ainsi obtenue est transformée en son chlorhydrate.

## Patentansprüche

1. Verfahren zur Herstellung von 1-[2-(2-Naphthyl)-ethyl)-4-(3-trifluormethylphenyl)-1,2,3,6-tetrahydropyridin der Formel (I) 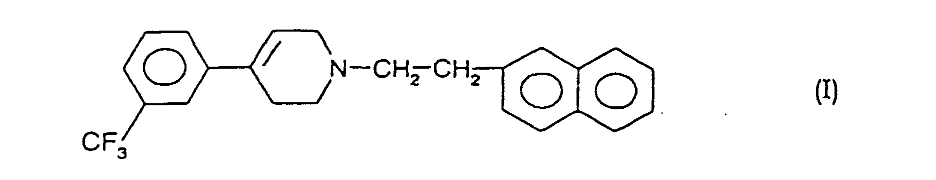 und seiner pharmazeutisch geeigneten Salze,
**gekennzeichnet durch**
Behandlung von 4-(3-Trifluormethylphenyl)-1,2,3,6-tetrahydropyridin der Formel (II) 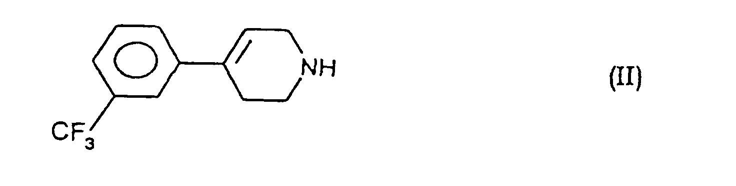 oder eines seiner Salze mit 2-(2-Bromethyl)-naphthalin der Formel (III) 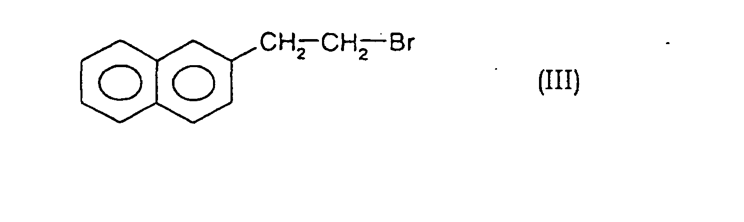 in Gegenwart einer Base bei einer Temperatur zwischen 20 °C und der Rückflusstemperatur des verwendeten Lösungsmittels.

2. Verfahren nach Anspruch 1, **dadurch gekennzeichnet, dass** die Verbindung (II) in Form des Hydrochlorids eingesetzt wird.

3. Verfahren nach Anspruch 2, **dadurch gekennzeichnet, dass** die Umsetzung in einem protischen oder aprotischen polaren Lösungsmittel am Rückfluss durchgeführt wird.

4. Verfahren nach Anspruch 3, **dadurch gekennzeichnet, dass** die Umsetzung in einem unter Aceton, Acetonitril und Wasser-Ethanol-Gemischen ausgewählten Lösungsmittel in Gegenwart einer unter Alkalihydroxiden und Alkalicarbonaten ausgewählten Base durchgeführt wird.

5. Verfahren zur Herstellung von 1-[2-(2-Naphthyl)-ethyl]-4-(3-trifluormethylphenyl)-1,2,3,6-tetrahydropyridin und seiner pharmazeutisch akzeptablen Salze,
**gekennzeichnet durch**
(a) Reduktion von 2-Naphthylessigsäure zu 2-Naphthylethanol und Umsetzung des so erhaltenen Produkts ohne Reinigung mit konzentrierter Bromwasserstoffsäure, anschließend
(b) Behandlung des so erhaltenen 2-(2-Bromethyl)-naphthalins mit 4-(3-Trifluormethylphenyl-1,2,3,6-tetrahydropyridin oder einem seiner Salze und
(c) Isolierung des 1-[2-(2-Naphthyl)-ethyl]-4-(3-trifluormethylphenyl)-1,2,3,6-tetrahydropyridins entweder in Form der Base, die gegebenenfalls in eines ihrer pharmazeutisch akzeptablen Salze umgewandelt wird, oder in Form eines seiner Salze, das gegebenenfalls unter Erhalt der freien Base neutralisiert wird.

6. Verfahren nach Anspruch 5, **dadurch gekennzeichnet, dass** in Stufe (a) als Reduktionsmittel Lithiumalanat verwendet wird.

7. Verfahren nach Anspruch 6, **dadurch gekennzeichnet, dass** die Reduktion in Tetrahydrofuran durchgeführt wird.

8. Verfahren nach einem der Ansprüche 5 bis 7, **dadurch gekennzeichnet, dass** in Schritt (b) das Hydrochlorid von 4-(3-Trifluormethylphenyl)-1,2,3,6-tetrahydropyridin eingesetzt wird.

9. Verfahren nach Anspruch 8, **dadurch gekennzeichnet, dass** die Umsetzung der Stufe (b) in einem protischen oder aprotischen polaren Lösungsmittel in Gegenwart einer Base durchgeführt wird.

10. Verfahren nach Anspruch 9, **dadurch gekennzeichnet, dass** das protische oder aprotische polare Lösungsmittel unter Acetonitril, Aceton und Ethanol-Wasser-Gemischen am Rückfluss und die Base unter Alkalihydroxiden und Alkalicarbonaten ausgewählt werden.

11. Verfahren nach Anspruch 10, **dadurch gekennzeichnet, dass** die Stufe (b) durch Behandlung von 2-(2-Bromethyl)-naphthalin in einem Ethanol-Wasser-Gemisch im Verhältnis 2:1 bis 1:1 (V/V) in Gegenwart eines Alkalihydroxids am Rückfluss durchgeführt wird.

12. Verfahren nach Anspruch 11, **dadurch gekennzeichnet, dass** als Alkalihydroxid Natriumhydroxid verwendet wird.

13. Verfahren nach Anspruch 11 oder 12, **dadurch gekennzeichnet, dass** in Stufe (c) die 1-[2-(2-Naphthyl)-ethyl]-4-(3-trifluormethylphenyl)-1,2,3,6-tetrahydropyridin-Base durch Filtration isoliert wird, die gegebenenfalls in eines ihrer pharmazeutisch akzeptablen Salze umgewandelt wird.

14. Verfahren nach Anspruch 13, **dadurch gekennzeichnet, dass** die so erhaltene Base in ihr Hydrochlorid umgewandelt wird.

## Claims

1. A process for the preparation of 1-[2-(naphth-2-yl)ethyl]-4-(3-trifluoromethylphenyl)-1,2,3,6-tetrahydropyridine of formula (I): 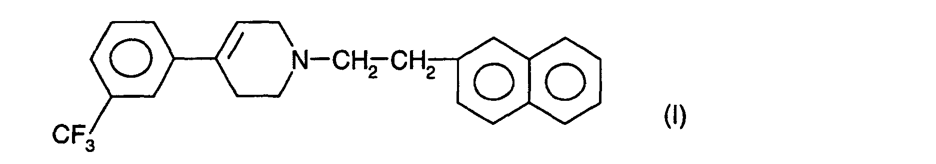 and its pharmaceutically acceptable salts, **characterized in that** 4-(3-trifluoromethylphenyl)-1,2,3,6-tetrahydropyridine of formula (II): 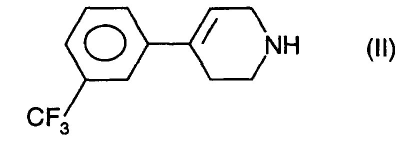 or one of its salts, is treated with 2-(2-bromoethyl)naphthalene of formula (III): 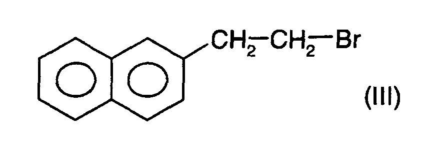 in the presence of a base and at a temperature between 20°C and the reflux temperature of the solvent employed.

2. A process according to claim 1 **characterized in that** the compound (II) is used in the form of the hydrochloride.

3. A process according to claim 2 **characterized in that** the reaction is carried out in a polar protic or aprotic solvent under reflux.

4. A process according to claim 3 **characterized in that** the reaction is carried out in a solvent selected from acetone, acetonitrile and water/ethanol mixtures, in the presence of a base selected from alkali metal hydroxides and carbonates.

5. A process for the preparation of 1-[2-(naphth-2-yl)ethyl]-4-(3-trifluoromethylphenyl)-1,2,3,6-tetrahydropyridine and its pharmaceutically acceptable salts, **characterized in that**:
(a) naphth-2-ylacetic acid is reduced to 2-(naphth-2-yl)ethanol and the resulting product is reacted, without purifying it, with concentrated hydrobromic acid;
(b) then the resulting 2-(2-bromoethyl)naphthalene is treated with 4-(3-trifluoromethylphenyl)-1,2,3,6-tetrahydropyridine or one of its salts; and
(c) the 1-[2-(naphth-2-yl)ethyl]-4-(3-trifluoromethylphenyl)-1,2,3,6-tetrahydropyridine is isolated either in the form of the base, which is optionally converted to one of its pharmaceutically acceptable salts, or in the form of one of its salts, which is optionally neutralized to give the free base.

6. A process according to claim 5 **characterized in that** lithium aluminum hydride is used as the reducing agent in step (a).

7. A process according to claim 6 **characterized in that** the reduction is carried out in tetrahydrofuran.

8. A process according to any one of claims 5 to 7 **characterized in that** 4-(3-trifluoromethylphenyl)-1,2,3,6-tetrahydropyridine hydrochloride is used in step (b).

9. A process according to claim 8 **characterized in that** the reaction of step (b) is carried out in a polar protic or aprotic solvent in the presence of a base.

10. A process according to claim 9 **characterized in that** the polar protic or aprotic solvent is selected from acetonitrile, acetone and ethanol/water mixtures under reflux, and the base is selected from alkali metal hydroxides and carbonates.

11. A process according to claim 10 **characterized in that** step (b) is carried out by treating 2-(2-bromoethyl)naphthalene in a 2/1 to 1/1 (v/v) ethanol/water mixture, in the presence of an alkali metal hydroxide, under reflux.

12. A process according to claim 11 **characterized in that** sodium hydroxide is used as the alkali metal hydroxide.

13. A process according to claim 11 or 12 **characterized in that**, in step (c), 1-[2-(naphth-2-yl)ethyl]-4-(3-trifluoromethylphenyl)-1,2,3,6-tetrahydropyridine base is isolated by filtration and optionally converted to one of its pharmaceutically acceptable salts.

14. A process according to claim 13 **characterized in that** the base obtained is converted to its hydrochloride.
